(19) Europäisches Patentamt / European Patent Office / Office européen des brevets

(11) **EP 3 875 093 A1**

(12) **EUROPEAN PATENT APPLICATION**
published in accordance with Art. 153(4) EPC

(43) Date of publication:
**08.09.2021 Bulletin 2021/36**

(51) Int Cl.:
*A61K 31/513* [(2006.01)]  *A61P 9/00* [(2006.01)]
*A61P 9/04* [(2006.01)]  *A61P 11/00* [(2006.01)]

(21) Application number: **19878056.1**

(22) Date of filing: **31.10.2019**

(86) International application number:
**PCT/JP2019/042924**

(87) International publication number:
**WO 2020/091014 (07.05.2020 Gazette 2020/19)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **02.11.2018 JP 2018207116**

(71) Applicant: **Furukawa Research Office Co., Ltd.
Tokyo 157-0066 (JP)**

(72) Inventor: **FURUKAWA, Satoru
Tokyo 157-0066 (JP)**

(74) Representative: **Brand Murray Fuller LLP
50 Eastcastle Street
London W1W 8EA (GB)**

(54) **HEART RATE DECREASING AGENT**

(57) The present invention addresses the problem of providing an agent for decreasing heart rate having fewer side effects, or a novel agent for suppressing an increase in heart rate during exercise. The heart rate can be decreased, in particular, palpitation, shortness of breath or fatigue associated with an increased heart rate can be prevented or ameliorated, and the increase in heart rate during exercise can be suppressed by ingesting a preparation containing orotic acid or a salt thereof as an active ingredient.

[Fig. 1]

## Description

### Technical Field

The present invention relates to an agent for decreasing heart rate, an agent for preventing or ameliorating palpitation, shortness of breath or fatigue associated with an increased heart rate, and an agent for suppressing an increase in heart rate during exercise, containing orotic acid or a salt thereof (hereinafter, may be referred to as "orotic acid group") as an active ingredient.

### Background Art

Orotic acid (also called 6-carboxyuracil, or vitamin B13) is an important intermediate in the pyrimidine nucleotide biosynthesis pathway. It is derived from dihydroorotic acid by dihydroorotic acid dehydrogenase and converted to orotidylic acid by orotate phosphoribosyltransferase (PRPP). Orotidylic acid is quickly further converted to uridine monophosphate (UMP), after which pyrimidine nucleotides such as uridine triphosphate and cytidine triphosphate are synthesized.

In recent years, research on the physiological effects of orotic acid has been pursued. For example, it is known that orotic acid has the effect of decreasing blood uric acid levels (Patent Document 1), orotic acid has the effect of improving endurance (Patent Documents 2 and 3), orotic acid has the effect of reducing oxygen consumption and energy consumption (Patent Document 3), and has the effect of activating the sympathetic nervous system, ameliorating drowsiness, raising body temperature, accelerating the breakdown of fat or maintaining focus (Patent Document 4).

On one hand, heart rate, despite being one of the important vital signs alongside blood pressure, respiratory rate, body temperature, and consciousness, had not received much attention until now, but has started gaining attention as one of the important biomarkers for cardiovascular disease, since the higher the heart rate is, the worse the prognosis is for cardiovascular disease, or the prognosis improves when the heart rate drops in heart failure, ischemic heart disease, or the like.

On the other hand, an agent for decreasing heart rate, containing 2-[N-(2,6-dichlorophenyl)-N-allylamino]-2- imidazoline or an acid addition salt thereof as an active ingredient is known (Patent Document 5). It is also known that β-receptor blockers are used as a heart rate control agent for patients with hypertension or atrial fibrillation. Furthermore, a composition for oral administration for suppressing an increase in heart rate during exercise is known, which contains a casein hydrolysate obtained by hydrolyzing animal milk casein, containing free amino acids and peptides as active ingredients, and having an average chain length of 2.1 or less in terms of the number of amino acid residues, or a mixture of the free amino acids and peptide mixture contained in the hydrolysate (Patent Document 6).

### Prior Art Documents

#### Patent Documents

Patent Document 1: Japanese unexamined Patent Application Publication No. 2011-98896
Patent Document 2: Japanese unexamined Patent Application Publication No. 2011-136907
Patent Document 3: Japanese unexamined Patent Application Publication No. 2012-246280
Patent Document 4: Japanese unexamined Patent Application Publication No. 2012-126683
Patent Document 5: Japanese unexamined Patent Application Publication No. 55-15481
Patent Document 6: International Publication No. WO 2016-182053

### Summary of the Invention

### Object to be Solved by the Invention

Although β-receptor blockers, which are widely used at present, have a large effect of decreasing the heart rate, they also have strong side effects, and need to be used carefully according to the condition of the patient to be administered. In addition, only the hydrolysate of a natural product as described in Patent Document 6 is known as an agent for suppressing an increase in heart rate during exercise, and the composition thereof is difficult to adjust. In view of such circumstances, the present invention addresses the problem of providing an agent for decreasing heart rate having fewer side effects, or a novel agent for suppressing an increase in heart rate during exercise.

**Means to Solve the Object**

As a result of extensive studies to solve the above problems, the present inventors have found that orotic acid group, which activate the sympathetic nervous system, have the effect of decreasing the heart rate, and that in cases where the heart rate usually increases during exercise and it is difficult to exercise, it is possible to suppress the increase in heart rate and exercise comfortably by taking orotic acid group, and completed the present invention.

That is, the present invention is as specified by the following matters.

(1) An agent for decreasing heart rate, comprising orotic acid or a salt thereof as an active ingredient.

(2) An agent for preventing or ameliorating palpitation, shortness of breath or fatigue associated with an increased heart rate, wherein the agent comprises orotic acid or a salt thereof as an active ingredient.

(3) The agent for preventing or ameliorating palpitation, shortness of breath or fatigue associated with an increased heart rate according to the above (2), wherein the palpitation or shortness of breath associated with an increased heart rate is caused by exercise, aging, a decrease in cardiopulmonary function or drinking.

(4) An agent for suppressing an increase in heart rate during exercise, comprising orotic acid or a salt thereof as an active ingredient.

(5) The agent according to any one of the above (1) to (4), which is for oral administration.

Other aspects of the present invention include the following:

(6) A method for decreasing the heart rate by administering an effective amount of orotic acid or a salt thereof to a subject.

(7) Orotic acid or a salt thereof used for decreasing the heart rate.

(8) Use of orotic acid or a salt thereof for producing an agent for decreasing heart rate.

(9) A method for preventing or ameliorating palpitation, shortness of breath or fatigue associated with an increased heart rate, by administering an effective amount of orotic acid or a salt thereof to a subject.

(10) Orotic acid or a salt thereof used for preventing or ameliorating palpitation, shortness of breath or fatigue associated with an increased heart rate.

(11) Use of orotic acid or a salt thereof for producing an agent for preventing or ameliorating palpitation, shortness of breath or fatigue associated with an increased heart rate.

(12) A method for suppressing an increase in heart rate during exercise by administering an effective amount of orotic acid or a salt thereof to a subject.

(13) Orotic acid or a salt thereof used for suppressing an increase in heart rate during exercise.

(14) Use of orotic acid or a salt thereof for producing an agent for suppressing an increase in heart rate during exercise.

(15) A method for administering the heart rate decreasing agent according to the above (1), the agent for preventing or ameliorating palpitation, shortness of breath or fatigue associated with an increased heart rate according to the above (2), or the agent for suppressing an increase in heart rate according to the above (4) by oral administration.

(16) Orotic acid or a salt thereof used for orally administering the heart rate decreasing agent according to the above (1), the agent for preventing or ameliorating palpitation, shortness of breath or fatigue associated with an increased heart rate according to the above (2), or the agent for suppressing an increase in heart rate according to the above (4).

(17) Use of orotic acid or a salt thereof for producing the heart rate decreasing agent according to the above (1), the agent for preventing or ameliorating palpitation, shortness of breath or fatigue associated with an increased heart rate according to the above (2), or the agent for suppressing an increase in heart rate according to the above (4) as an orally administered agent.

**Effect of the Invention**

Using the heart rate decreasing agent of the present invention allows to safely and efficiently decrease the heart rate, and to improve the prognosis of heart failure and ischemic heart disease.

Using the agent for preventing or ameliorating palpitation, shortness of breath or fatigue associated with an increased heart rate of the present invention allows to produce no, or alleviate the onset of symptoms of, palpitation, shortness of breath, fatigue, and the like caused by exercise, aging, a decrease in cardiopulmonary function, drinking, or the like, or to ameliorate such symptoms.

Moreover, using the agent for suppressing an increase in heart rate during exercise of the present invention allows to suppress the increase in heart rate and continue to exercise comfortably even in the cases where exercising is usually difficult due to the heart rate increasing during exercise.

In addition, the heart rate is controlled by the balance of the autonomic nervous system, and a decrease in heart rate is a phenomenon generally occurring when the parasympathetic nervous system is dominant. Therefore, an effect on other phenomena occurring when the parasympathetic nervous system is dominant, for example, the promotion of gastric acid and saliva secretion, the promotion of intestinal peristalsis, and sleep induction, can be expected, and the agent may be effective as a digestive agent, laxative, sleep inducer, and the like.

It may also be effective in alleviating symptoms in which the sympathetic nervous system is dominant such as stress and tension.

**Brief Description of Drawings**

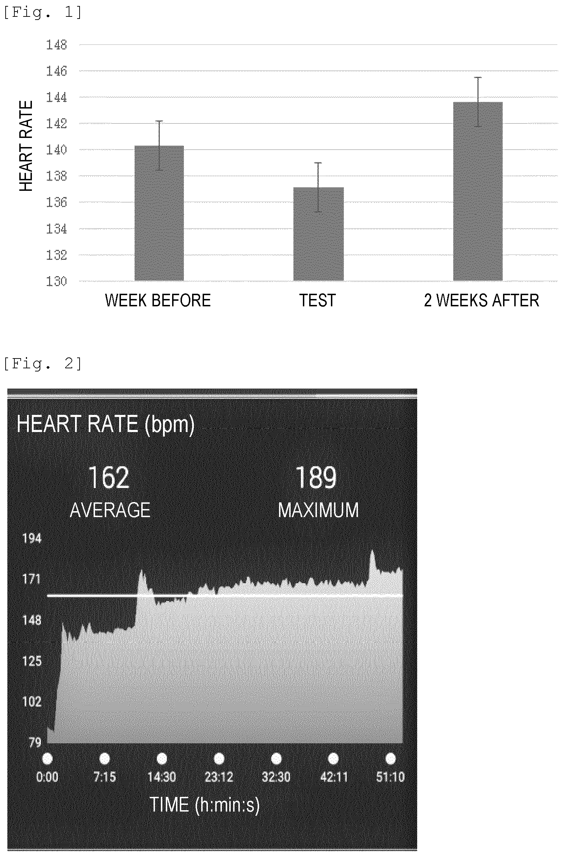

[Figure 1] Figure 1 shows the results of measuring the heart rate when training after ingesting orotic acid in 12 test subjects who go training daily in a hypoxic room of a sports club (Example 2). For comparison, the heart rate when training without ingesting orotic acid one week before and one week after the training was also measured, and the results are also shown. The vertical axis indicates the heart rate, and the horizontal axis indicates the time of the test when training after ingesting orotic acid, and when training without ingesting orotic acid one week before and two weeks after. The heart rate is the average value of the 12 test subjects.

[Figure 2] Figure 2 shows the results of measuring the variation of heart rate over time when a test subject, a male marathon runner in his thirties, did a practice run without ingesting orotic acid (Comparative Example of Example 3).

[Figure 3] Figure 3 shows the results of measuring the variation of heart rate over time when a test subject, a male marathon runner in his thirties, did a full marathon after ingesting orotic acid (Example 3).

[Figure 4] Figure 4 shows the results of measuring the variation of heart rate over time when a test subject, a male marathon runner in his twenties, did a 30 km run without ingesting orotic acid (Comparative Example of Example 4).

[Figure 5] Figure 5 shows the results of measuring the variation of heart rate over time when a test subject, a male marathon runner in his twenties, did a full marathon after ingesting orotic acid (Example 4).

[Figure 6] Figure 6 shows the results of measuring the variation of heart rate over time when a test subject, a male marathon runner in his twenties, did a half marathon without ingesting orotic acid (Comparative Example of Example 5).

[Figure 7] Figure 7 shows the results of measuring the variation of heart rate over time when a test subject, a male marathon runner in his twenties, did a full marathon after ingesting orotic acid (Example 5).

**Mode of Carrying Out the Invention**

The heart rate decreasing agent, the agent for preventing or ameliorating palpitation, shortness of breath or fatigue associated with an increased heart rate, or the agent for suppressing an increase in heart rate during exercise of the present invention (hereinafter, referred to as "heart rate decreasing agent and the like") is not limited, as long as it contains orotic acid or a salt thereof as an active ingredient. The above orotic acid is also called 6-carboxyuracil, but according to the IUPAC nomenclature, it is a kind of heterocyclic aromatic compound represented by "1,2,3,6-tetrahydro-2,6-dioxo-4-pyrimidinecarboxylic acid". In the present invention, the heart rate decreasing agent includes an agent decreasing the heart rate, as well as an agent capable of suppressing an increase in heart rate (maintaining the current condition) or reducing an increase in heart rate (there is an increase, but less than the increase when the agent is not ingested) when ingested, heart rate which would have increased if the agent was not ingested.

In addition, in the present invention, the agent for suppressing an increase in heart rate during exercise is an agent capable of reducing the increase from the normal heart rate when exercise is performed after ingesting the agent as compared with the increase from the normal heart rate when exercise is performed without ingesting the agent.

For example, bacteria belonging to the genus Corynebacterium having the ability to produce orotic acid can be cultured to produce and accumulate orotic acid in the culture, and using a production method for collecting this (see Japanese Patent Publication No. 7-10235) and a fermentation method using microorganisms, orotic acid can be produced and accumulated in the culture solution. Orotic acid can then be purified and collected from the culture by using a known conventional purification method such as a precipitation method or chromatography using an ion exchange resin or activated charcoal. Orotic acid can also be prepared by using a known chemical synthesis method or the like. Furthermore, a commercial product can also be used.

Examples of the orotic acid group include a free orotic acid (free form) and a salt of orotic acid, but a free orotic acid (free form) is preferable.

Examples of the salt of orotic acid include an acid addition salt, a metal salt, an ammonium salt, an organic amine addition salt, and an amino acid addition salt. The acid addition salt includes an inorganic acid salt such as a hydrochloride, a sulfate, a nitrate and a phosphate, and an organic acid salt such as an acetate, a maleate, a fumarate, a citrate, a malate, a lactate, an $\alpha$-ketoglutarate, a gluconate, and a caprylate. Examples of the metal salt include an alkali metal salt such as a sodium salt and a potassium salt, an alkaline earth metal salt such as a magnesium salt and a calcium salt, an aluminum salt, and a zinc salt. Examples of the ammonium salt include a salt of ammonium and tetramethylammonium. Examples of the organic amine addition salt include a salt of triethylamine, pyridine, morpholine, and piperidine. Examples of the amino acid addition salt include a salt of glycine, phenylalanine, lysine, aspartic acid, and glutamic acid.

As the heart rate decreasing agent and the like of the present invention, orotic acid or a salt thereof can be administered as it is, but it is preferable to mix it with a carrier or the like and provide it as various compositions: medicaments, foods, drinks, and supplements, as needed.

The salt of orotic acid provided as the medicament is not limited as long as it is a pharmaceutically acceptable salt, but when the orotic acid salt is dissolved in water, examples thereof include a choline salt, a lysine salt, an arginine salt, and an ornithine salt of which the aqueous solution is neutral to weakly acidic and which are less likely to precipitate or deposit during storage, and a water-soluble salt thereof is preferable in the case of a beverage. Moreover, when ingested as a capsule or tablet, it does not require to be water-soluble, and therefore examples thereof include a poorly soluble metal salt such as a sodium salt, a potassium salt, a magnesium salt, a calcium salt and an ammonium salt. Orotic acid can also form a salt with carnitine and be solubilized. The carnitine salt (L-carnitine orotate) has a good solubility, but since its aqueous solution has a low pH, it can be used by making the pH weakly acidic by adding a purine base such as guanosine or a basic amino acid as needed.

When the heart rate decreasing agent and the like of the present invention is provided as a medicament, orotic acid or a salt thereof is used as it is, or is prepared in various preparation forms which can be administered orally or parenterally, systemically or topically by various known methods, by using an appropriate additive (such as a carrier, an excipient, a diluent, a binder, a lubricant, a disintegrant or disintegrant aid, a solubilizer, a stabilizer, a preservative, an antiseptic, an antioxidant, a microbial inhibitor, a bulking agent, a thickener, an emulsifier, a dispersant, a suspending agent, and a buffer) which is pharmaceutically acceptable and appropriately selected according to the dosage form.

For example, in the case of a liquid preparation such as a syrup suitable for oral administration, water, a sugar such as sucrose, sorbitol and fructose, a glycol such as polyethylene glycol and propylene glycol, an oil such as sesame oil, olive oil and soybean oil, an antiseptic such as a p-hydroxybenzoic acid ester, a flavor such as strawberry flavor and peppermint, and the like can be added for formulation.

In the case of a tablet, a powder, a granule, and the like suitable for oral administration, an excipient such as a sugar such as lactose, refined sugar, glucose, sucrose, mannitol, and sorbitol, a starch such as potato, wheat, and corn, an inorganic substance such as calcium carbonate, calcium sulfate, sodium hydrogen carbonate and sodium chloride, and a plant powder such as powdered glycyrrhiza and powdered gentian; a disintegrant such as a starch, agar, gelatin powder, crystalline cellulose, carmellose sodium, carmellose calcium, calcium carbonate, sodium hydrogen carbonate, and sodium alginate; a lubricant such as magnesium stearate, talc, a hydrogenated vegetable oil, macrogol, and silicon oil; a binder such as polyvinyl alcohol, hydroxypropyl cellulose, methyl cellulose, ethyl cellulose, carmellose, gelatin, and starch paste; a surfactant such as a fatty acid ester; a plasticizer such as glycerin and the like can be added for formulation.

In addition, an additive generally used in foods and drinks, such as a sweetener, a colorant, a preservative, a thickening stabilizer, an antioxidant, a color former, a bleaching agent, a fungicide, a gum base, a bittering agent, an enzyme, a brightening agent, an acidulant, a seasoning, an emulsifier, a fortifier, a manufacturing agent, a flavor, and a spice extract may be added to a preparation suitable for oral administration.

The form of administration of the preparation can include oral administration or parenteral administration such as intravenous, intraperitoneal or subcutaneous administration, but oral administration is more preferable. The dosage form to be administered may be either an oral agent such as a tablet, a powder, a granule, a pill, a suspension, an emulsion, an infusion/decoction, a capsule, a syrup, a liquid, an elixir, an extract, a tincture, or a fluid extract, or a parenteral agent such as an injection (for example, a subcutaneous injection, an intravenous injection, an intramuscular injection, or an intraperitoneal injection), a drip, a suppository (for example, a rectal suppository or a vaginal suppository), an inhalant, a transdermal/transmucosal absorbent, an ointment, or a patch, but an oral agent is more preferable, and particularly, an agent for administration in the oral cavity is preferable.

Examples of the site for administering in the oral cavity include the lips, the movable oral mucosa, the non-movable oral mucosa, and the teeth.

The lips are divided into the upper lip and the lower lip, and are the area surrounding the opening of the oral cavity. The movable oral mucosa is a mucous membrane in the oral cavity which is movable by somatic nerves, and includes, for example, the mucous membrane behind the lips to the nasolabial fold or the lips to the mental protuberance, the buccal mucosa, and the tongue.

The non-movable oral mucosa is a fixed mucous membrane in the oral cavity which cannot be moved by somatic nerves, and includes, for example, the gingiva, the hard palate or soft palate mucosa, and the mucous membrane of the oral floor.

Teeth are hard structures which are planted on the jaw and play an important role mainly in eating.

When the heart rate decreasing agent and the like of the present invention is formulated as an agent for administration in the oral cavity, a dosage form with which the active ingredient remains on the lips, movable oral mucosa, non-movable oral mucosa, or teeth in the oral cavity for a relatively long time can be used, such as a gargle preparation such as a solution, a suspension, an emulsion, a granule, a fine granule, a powder or a gel; an application preparation such as an ointment, a cream, a gel, a viscous agent, an adhesive, a granule, a fine granule or a powder; a pasting preparation such as a tape, a cataplasm, or a film; an aerosolized agent such as an external aerosol spray, or a pump spray; a lozenge, a tablet, a buccal preparation, a sublingual formulation, or a gum agent, which are publicly known.

For a solution, a suspension, an emulsion, a cream, a gel, and a viscous agent, the dosage form is a continuous layer of water, an aqueous solvent which can be mixed with water in an unlimited ratio, and a mixed solvent thereof, and the appearance may be transparent, translucent or opaque. It is usually fluid, but some creams, viscous agents, and gels also have shape retention properties. These agents can be prepared using known components, and examples of such components include a surfactant such as an anionic surfactant such as an N-acyl amino acid salt, an N-acyl taurine salt, and an alkylsulfate ester salt, an amphoteric surfactant such as an imidazoline-based amphoteric surfactant and a betaine surfactant, a nonionic surfactant such as a sorbitan-based surfactant, a glycerin fatty acid ester, a polyglycerol fatty acid ester, a propylene glycol fatty acid ester, an alkyl glycoside, a tween-based surfactant, a polyoxyethylene glycerin fatty acid ester, a polyoxyethylene polyglycerol fatty acid ester, a polyoxyethylene propylene glycol fatty acid ester, a polyoxyethylene hydrogenated castor oil derivative, a sucrose fatty acid ester, a polyoxyethylene alkyl ether, a Pluronic surfactant, and an alkanolamide, and a naturally occurring surfactant such as a lecithin and a saponin; a fat or oil such as vegetable oil, animal oil, hydrogenated oil; a hydrocarbon oil such as liquid paraffin, squalane, and petrolatum; a higher fatty acid such as lauric acid, stearic acid, and oleic acid; a wax such as beeswax, lanolin and derivatives thereof; a monoalkyl fatty acid ester; a polyhydric alcohol fatty acid ester; an alcohol such as a lower alcohol such as ethyl alcohol and butyl alcohol, a higher alcohol such as lauryl alcohol, stearyl alcohol, behenyl alcohol, and batyl alcohol, and other alcohols such as lanolin alcohol, cholesterol, and phytosterol; a polyhydric alcohol such as ethylene glycol, propylene glycol and glycerol; a polyhydric alcohol polymer such as dipropylene glycol, diglycerol and polyglycerol; a sugar alcohol such as sorbitol, maltitol, mannitol, and erythritol; a mucopolysaccharide such as chondroitin sulfate, hyaluronic acid, and heparin; an organic compound such as sodium lactate, pyrrolidone carboxylate, and collagen; a moisturizing agent such as a plant extract; and a thickener (including a gelling agent) or a dispersant such as a cellulose derivative such as hydroxyethyl cellulose, hydroxypropyl cellulose, carboxymethyl cellulose, and hydroxyethyl carboxymethyl cellulose, a natural polymer such as acacia, carrageenan, tragacanth gum, gum karaya, gum arabic, guar gum, agar, an alginic acid, a collagen hydrolysate, gelatin, and a casein protein, a microbial polymer such as gellan gum, and xanthan gum, a synthetic polymer such as polyvinyl alcohol, sodium polyacrylate, carboxyvinyl polymer, and polyvinylpyrrolidone, and an inorganic thickener such as aluminum stearate gel, anhydrous silicic acid, calcium phosphate, and calcium carbonate. In addition, a flavor such as anethol, eugenol, methyl salicylate, limonene, ocimene, n-decyl alcohol, citronellol, α-terpineol, methyl acetate, citronellyl acetate, methyl eugenol, cineol, linalool, ethyl linalool, thymol, lemon oil, orange oil, sage oil, rosemary oil, cinnamon oil, perilla oil, wintergreen oil, clove oil, eucalyptus oil, pimento oil, d-camphor, d-borneol, fennel oil, cinnamon oil, cinnamaldehyde, peppermint oil, and vanillin; a sweetener such as sodium saccharin, acesulfame potassium, stevioside, neohesperidin dihydrochalcone, perillartine, thaumatin, aspartylphenylalanine methyl ester, and p-methoxy cinnamic aldehyde; an antiseptic such as a p-hydroxybenzoate ester, sodium benzoate, phenoxyethanol, and alkyldiaminoethylglycine hydrochloride; a legal pigment such as Blue No. 1, Yellow No. 4, Red No. 202, and Green No. 3, a mineral pigment such as ultramarine, rich ultramarine and prussian blue, and a colorant (including an opacifier) such as titanium oxide; a pH adjuster such as citric acid, phosphoric acid, malic acid, pyrophosphoric acid, lactic acid, tartaric acid, glycerophosphoric acid, acetic acid, nitric acid, a chemically acceptable salt thereof, and sodium hydroxide; and other pharmaceutical components can be added as needed.

Granules, fine granules and powders are powdered solid formulations, and lozenges, tablets, buccal preparations, and sublingual formulations are lumpy solid formulations. The granules, fine granules and powders can be prepared by simply mixing substances in powdered form, or by adjusting the particle size to a predetermined size by a granulation step or the like. They can also be prepared by drying the above solutions, dispersants, suspending agents and the like by spray drying or the like. When preparing these dosage forms, excipients, disintegrants, binders and the like are added as needed. In addition, since they are used by dissolving them in the mouth, sweeteners, flavoring agents and flavors can be added as needed. Examples of the excipient include refined sugar, lactose, mannitol, crystalline cellulose, starch, alginic acid, calcium phosphate, and calcium carbonate. Examples of the disintegrant include a cellulose derivative such as carboxymethyl cellulose, carboxymethyl cellulose calcium and crystalline cellulose, and starch. Examples of the binder include a cellulose derivative such as hydroxypropyl cellulose and methyl cellulose, acacia, gelatin, dextrin, and polyvinylpyrrolidone. Lozenges, tablets, buccal preparations, and sublingual formulations can be prepared by using a preparation obtained by mixing powdery raw materials and performing granulation, or a preparation obtained by the same method as the above granules, fine granules and powders, and molding it into a predetermined shape. The surface of the molded product can also be coated after molding as long as it does not interfere with mastication and dissolution in the mouth.

Ointments can be prepared by mixing with a lanolin, Plastibase, white petrolatum, paraffin, beeswax, an absorption ointment, polyethylene glycol, propylene glycol, glycerin, stearyl alcohol, stearic acid, glycerogelatin, vegetable oil, cacao butter, and the like.

Tapes and cataplasms are sheet-shaped dosage forms composed of a support, an applicator and the like. The substances used in tapes and cataplasms can be used as the support, but it is preferable to select a substance of which the shape eventually disappears by dissolving, decomposing, or dispersing during use, since it is used in the mouth. Examples of such a substance include polyacrylic acid, hydroxypropyl cellulose, and hypromellose phthtalate.

Although the mechanism of action of the heart rate decreasing agent of the present invention is unclear, it can decrease the heart rate without interfering with other functions of the atrium, and therefore is effective for the improvement of symptoms such as palpitation and shortness of breath associated with tachycardiac atrial fibrillation, the prevention of heart failure/tachycardia-induced cardiomyopathy, the improvement of prognosis and QOL of chronic heart failure, the treatment of hypertension, the control of symptoms associated with myocardial ischemia, the prevention of serious cardiovascular events such as acute myocardial infarction and death, and the like. In particular, it is effective as an agent for preventing and/or ameliorating palpitation, shortness of breath or fatigue associated with an increased heart rate caused by exercise, aging, a decrease in cardiopulmonary function, drinking, or the like.

When comparing a certain point of time with a certain point of time thereafter, an "increase in heart rate" refers to when the heart rate at a certain point of time thereafter is higher than the heart rate at a certain point of time, and is relative. Specific examples include a heart rate higher after exercise than before exercise, if it is due to exercise, a heart rate after a certain time in the past higher than in the past for a heart rate in the same condition, if it is due to aging, a heart rate when cardiopulmonary function is decreased higher than before when cardiopulmonary function has decreased compared to before, if it is due to a decrease in cardiopulmonary function, and a heart rate higher after drinking compared to before drinking, if it is due to drinking.

The agent for suppressing an increase in heart rate during exercise of the present invention is an agent for suppressing an increase in heart rate associated with the load on the body due to exercise during exercise or competition such as walking, jogging, running, marathon, swimming, cycling, aerobics, boating, baseball, tennis, table tennis, soccer, basketball, volleyball, dance, archery, cycling, bicycle racing, skiing, skating, skateboarding, trekking, mountain climbing, diving, skydiving, motorcycle racing, and bicycle racing.

The subject taking the above suppressing agent is a person who engages in exercise for the purpose of health improvement or the prevention or improvement of lifestyle-related diseases or as a hobby (hereinafter, referred to as "general exercising person") or a competitive exercising person called an athlete. General exercising persons include potential targets for exercise (hereinafter, referred to as "potential exercising persons") who, while not particularly habitually exercising at present, want to make it a habit or think that it should become a habit.

Exercise intensity is an index representing the physical load during exercise, and can be calculated, for example, by the following Karvonen formula using the heart rate.

$$\text{Exercise intensity (\%)} = (\text{heart rate during exercise} - \text{heart rate at rest}) / (\text{maximum heart rate} - \text{heart rate at rest}) \times 100$$

While the maximum heart rate can be measured individually, it can also be calculated as an estimate by the following formula:

$$\text{Maximum heart rate} = 220 - \text{age}$$

For elderly people, it can also be calculated by the following formula:

$$\text{Maximum heart rate (for elderly people)} = 207 - (\text{age} \times 0.7)$$

The above exercise intensity is 0% at rest, less than 20% when very light, 20 to 39% when light, 40 to 59% when moderate, 60 to 84% when high, and 85% or more when very high (see Keio University's Sports Medical Research Center Bulletin, p. 33-39, 1999).

When this is subjectively expressed, less than 40% is light exercise, 40% or more and less than 80% is slightly hard exercise to very hard exercise, and 80% or more is exercise near the limit.

For a general exercising person or a potential exercising person, the discomfort associated with an increase in heart rate during exercise may constitute a barrier to exercise consistently. In particular, the more a person gets old, the more the sensitivity of exercise intensity to an increase in pulse rate increases, and the more he/she feels that exercise is hard at a low heart rate (see the Ministry of Health, Labour and Welfare's Study Panel Report on Revision of Exercise Standards and Exercise Guidelines, March 2013). Therefore, by taking the agent for suppressing an increase in heart rate during exercise of the present invention before exercise, it is possible for general exercising persons to maintain their exercise habits comfortably even at an old age, and it is a trigger for potential exercising persons to start or restart exercise.

Moreover, for those in need of rehabilitation, it is also possible to reduce the discomfort associated with an increase in heart rate due to the physical load during rehabilitation by taking the agent for suppressing an increase in heart rate during exercise of the present invention before rehabilitation.

For athletes, especially in competitions during which a state of increased heart rate due to physical load continues for a long period of time, it may lead to improvement in endurance if the degree of increase in heart rate can be suppressed.

The exercise intensity when ingesting the agent for suppressing an increase in heart rate during exercise of the present invention is not limited, and is any exercise intensity exceeding 0%. In particular, it is preferably ingested when exercising at an exercise intensity of 80% or less, more preferably ingested when exercising at an exercise intensity of 60% or less, and further preferably ingested when exercising at an exercise intensity of 10 to 40%.

The duration of exercise is not limited, and while the agent for suppressing an increase in heart rate during exercise of the present invention can be applied to exercise from several seconds to several hours, it can be suitably used for exercise sustained for at least 5 to 10 minutes.

It is preferable to ingest the heart rate decreasing agent and the like of the present invention on a regular basis, but when partaking in exercise, drinking, or the like expected to increase the heart rate, it is preferable to ingest it within 3 hours before, and further preferable to ingest it within 2 hours before.

Moreover, when exercising for 30 minutes or more, preferably 1 hour or more, the agent can be ingested not only before exercise but also during exercise and after exercise.

When the heart rate decreasing agent and the like of the present invention is used as a medicament, the dose can be appropriately determined according to the age, sex, body weight, and degree of symptoms of the subject of administration, the administration method, and the like, but usually an amount of 10 mg to 10 g, preferably 50 mg to 5 g, more preferably 100 mg to 1 g of orotic acid or a salt thereof per day for adults is administered once or several times a day.

When the heart rate decreasing agent and the like of the present invention is provided as a medicament, the content of the active ingredient orotic acid or a salt thereof is appropriately selected according to the type of medicament, the effect expected by the administration of the medicament, and the like, but is usually 0.01 to 100 mass%, preferably 0.05 to 100 mass%, and more preferably 0.1 to 100 mass%.

The heart rate decreasing agent and the like of the present invention may also be added to foods, drinks or supplements as an additive for foods, drinks or supplements. In this case, the amount to be added may be appropriately determined in consideration of the conventional intake of the object food, drink or supplement, the form of the food or drink, the efficacy/effect, the taste, the palatability, the cost, and the like, but is usually added to obtain a content of the active ingredient orotic acid or a salt thereof in a food, drink or supplement of 0.01 to 100 mass%, preferably 0.05 to 100 mass%, and more preferably 0.1 to 100 mass%.

When the heart rate decreasing agent and the like of the present invention is used as an additive for foods, drinks or supplements, various proteins, sugars, fats, trace elements, vitamins, organic acid salts such as citric acid and acetic acid, and the like may be contained as other components as long as they do not undermine the effects of the present invention. In addition, depending on the type of food, drink or supplement to be added, an additive that is acceptable and commonly used in foods, drinks or supplements, for example, a sweetener such as aspartame and stevia, an acidulant such as citric acid, malic acid and tartaric acid, and an excipient such as a dextrin and a starch, but also a colorant, a flavor, a bittering agent, a buffer, a thickening stabilizer, a gelling agent, a stabilizer, a gum base, a binder, a diluent, an emulsifier, a dispersant, a suspending agent, an antioxidant, a preservative, an antiseptic, a fungicide, a color former, a bleaching agent, a brightening agent, an enzyme, a seasoning, and a spice extract may be contained.

Examples of the food and drink to which the heart rate decreasing agent and the like of the present invention is added include a beverage such as a tea beverage, a beer-based beverage, coffee, mineral water, and a milk beverage (including a liquid concentrate and a powder for preparations of these beverages); a carbohydrate-containing product such as rice, noodles, bread, and pasta; various confectioneries such as a western confectionery such as cookies and cake, a Japanese confectionery such as a steamed bread and yokan, a cold confectionery such as candy, gum, pudding, and jelly, and an ice confectionery; processed seafood and livestock food such as kamaboko, chikuwa, hamburger, ham, and sausage; a dairy product such as processed milk, fermented milk, yogurt, butter, and cheese; oil, fat, and processed oil and fat such as margarine, mayonnaise, shortening, whipped cream, and dressing, and a seasoning such as a sauce and a dipping sauce.

The supplement to which the heart rate decreasing agent and the like of the present invention is added is not limited as long as it is a so-called health food such as a functional food, a dietary supplement, a health supplement, a fortified food, or a nutrient controlling food, but examples thereof include a base supplement that supplements the vitamins (for example, vitamin B1, vitamin B2, and multivitamin), minerals (for example, iron and zinc), amino acids or dietary fiber, DHA, EPA, and the like needed by the body, a health supplement used for health maintenance or beauty containing isoflavone, royal jelly, propolis, sesamine, catechin, and the like, and an optional supplement used for the recovery of health containing turmeric, maca, blueberry, glucosamine, and the like.

The shape of the food, drink or supplement obtained by adding the heart rate decreasing agent and the like of the present invention is not limited as long as it can be ingested by mammals and is suitable for consumption, but examples thereof include solid, liquid, semi-liquid, granular, granulated, powdered, capsular, creamy, pasty, and jelly.

The food or drink obtained by adding the heart rate decreasing agent and the like of the present invention includes a health food, a functional food, a food for specified health use, a dietary supplement, and a food for the sick. In particular, it is preferable to provide it as a food suitable for consumers who are expecting the improvement of symptoms such as palpitation and shortness of breath due to tachycardiac atrial fibrillation, the prevention of heart failure/tachycardia-induced cardiomyopathy, the improvement of prognosis and QOL of chronic heart failure, the treatment of hypertension, the control of symptoms associated with myocardial ischemia, the prevention of serious cardiovascular events such as acute myocardial infarction and death, and the like by decreasing the heart rate, that is, as a food for specified health use.

In addition, the heart rate decreasing agent and the like of the present invention can be used as a material for fermented foods.

The intake of a food, drink or supplement obtained by adding the heart rate decreasing agent and the like of the present invention may be an amount at which a heart rate decreasing effect can be exerted, and is not limited, but usually, the daily intake for adults is the amount at which orotic acid or a salt thereof is 10 mg to 10 g, preferably 50 mg to 5 g, and more preferably 100 mg to 1 g.

**Examples**

Hereafter, the present invention will be further described specifically with Examples, but the present invention is not limited to these Examples.

Example 1

Tablets consisting of orotic acid were orally ingested and the variation in heart rate was measured before and after oral ingestion. The test subject was a 62-year-old man. Although he was told at his medical checkup that his cholesterol was slightly high, he had not taken any medication and had no preexisting condition. His blood pressure was in the normal range, and he had never been diagnosed with a blood pressure problem in the past. His heart rate was slightly high at about 89 to 92 beats /min, and rarely fell below 86 beats /min even at rest.

Since the heart rate quickly increases due to exercise and tension or the like, the measurement day was spent as much as possible doing desk work and the like, and the measurement was made under stable conditions after sitting on a chair for at least several minutes.

He was drinking 2 cans of beer and 2 to 3 double glasses of shochu daily. His heart rate after drinking was increasing to about 105 to 115 beats /min (120 beats /min in rare cases), and he was feeling drunk and mild palpitation. He was also feeling sleepy on some days.

The male test subject ingested a tablet containing 200 mg of orotic acid by oral administration, and the measurement of his heart rate was started. His heart rate gradually decreased from about 2 hours after ingestion of orotic acid, and was 80 beats /min or less 4 hours later, then stabilized in the 70s beats /min to the lower 80s beats /min. With a decreased heart rate after ingestion, he had the impression that his head was more refreshed and no longer felt lethargic like he always felt as compared with his condition before ingestion of orotic acid.

He drank the usual amount of beer and shochu after the decrease in heart rate, but his heart rate only rose to 90 to the lower 100s beats /min, and he unusually felt almost no drunkenness or palpitation.

The above results are summarized in Table 1.

[Table 1]

|  | Heart rate at the start of measurement (before orotic acid ingestion) | Heart rate 4 to 5 hours after orotic acid ingestion | Symptoms | Heart rate 1 to 2 hours after drinking | Symptoms |
|---|---|---|---|---|---|
| Placebo | 89 to 93 beats/min | 88 to 93 beats/min | Languor | 105 to 115 beats/min | Slight drunkenness Mild palpitation Drowsiness |
| Orotic acid ingestion | | 77 to 82 beats/min | Clear head No languor | 85 to 103 beats/min | No drunkenness nor palpitation |

From the results in Table 1, a decrease in heart rate was clearly observed with the intake of orotic acid, which also improved the palpitation and fatigue that were felt on a daily basis.

Example 2

Twelve test subjects who go training daily in a hypoxic room of a sports club were used to verify the effect of orotic acid on decreasing the heart rate during training.

Each test subject ingested two chewable tablets containing 200 mg of orotic acid by dissolving them in the oral cavity 2 hours before training.

The exercise intensity of each person was set to obtain a heart rate of about 30% of the maximum heart rate + 20 bpm. The exercise intensity was calculated by the above Karvonen formula. This exercise intensity corresponds to a rating of perceived exertion (Borg scale) of 11 to 13, and is a value close to the aerobic threshold.

Each test subject had their heart rate measured every 10 minutes and exercised for a total of 40 minutes.

For comparison, the heart rate when exercising in the same manner as in the test but without ingesting orotic acid was measured on the week before and after performing the test. The results are shown in Figure 1.

The vertical axis of Figure 1 indicates the heart rate. The heart rate value represents the average value of the 12 test subjects.

As is clear from Figure 1, a decrease in heart rate was observed when exercising after ingesting orotic acid (test) as compared with when orotic acid was not ingested (previous week and 2 weeks later).

In addition, after the test, a survey was conducted where each test subject indicated whether it was harder or easier than the usual exercise without ingesting orotic acid on a 10-point scale. The results are shown in Table 2. On the 10-point scale, 5 indicates that it is the same as usual, higher than 5 indicates that it is easier than usual, and smaller than 5 indicates that it is harder than usual.

[Table 2]

| Rating | 1 | 2 | 3 | 4 | 5 | 6 | 7 | 8 | 9 | 10 |
|---|---|---|---|---|---|---|---|---|---|---|
| Number of responses |  |  |  |  | 1 | 2 | 5 | 3 | 1 |  |

According to Table 2, many felt that ingesting orotic acid made exercising easier than usual.

Example 3

A male marathon runner in his thirties was used as a test subject to verify the heart rate decreasing effect during a marathon.

The test subject ran for 52 minutes and 50 seconds at an average pace of 04:44 min/km without ingesting orotic acid as a practice run, and his heart rate during that time was measured by Garmin's GPS fitness tracker. The results are shown in Table 3 and Figure 2.

[Table 3]

|  | Total time (ratio) |
|---|---|
| Heart rate zone 5 (heart rate of 169 beats or more) | 18 min 55 s (36%) |
| Heart rate zone 4 (150-168 beats) | 22 min 40 s (43%) |

(continued)

|  | Total time (ratio) |
|---|---|
| Lower heart rate (132-149 beats) | 10 min 54 s (20%) |

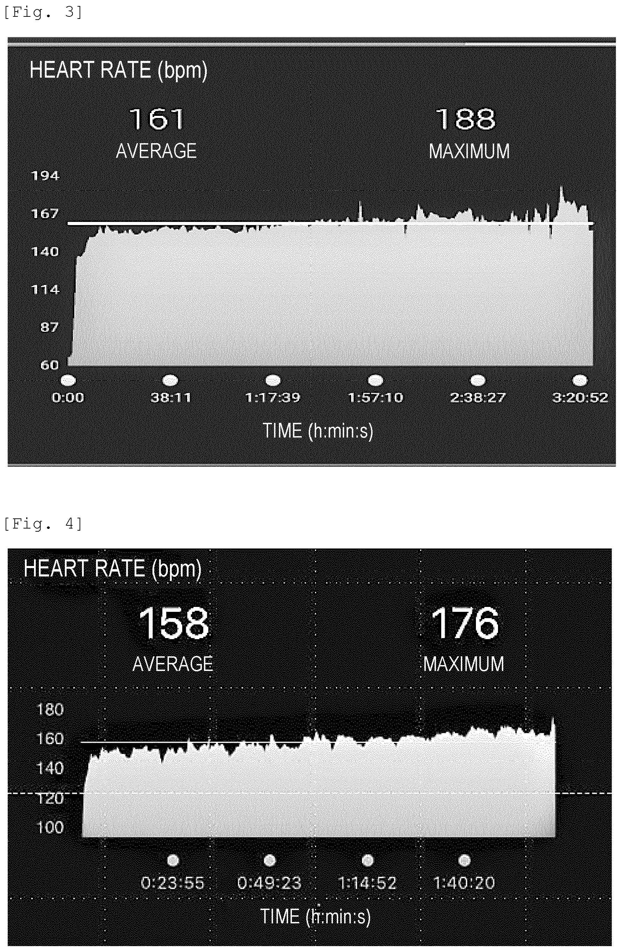

On the day of the marathon race (full marathon), the subject ingested two chewable tablets containing 200 mg of orotic acid by dissolving them in the oral cavity one hour before the start. Then, he participated in the race and completed it in 3 hours 24 minutes and 33 seconds at an average pace of 04:48 min/km. His heart rate during that time was measured in the same manner. The results are shown in Table 4 and Figure 3.

[Table 4]

|  | Total time (ratio) |
|---|---|
| Heart rate zone 5 (heart rate of 169 beats or more) | 23 min 55 s (12%) |
| Heart rate zone 4 (150-168 beats) | 2 h 54 min 0 s (85%) |
| Lower heart rate (up to 149 beats) | 6 min 7 s (3%) |

From Tables 3 and 4, it was found that the average pace during the marathon and practice was almost the same, and that the heart rate while running clearly decreased with the ingestion of orotic acid, even though the marathon was a longer distance and time.

In addition, according to Figures 2 and 3, while the overall average heart rate was 162 beats and 161 beats, the increase in heart rate was clearly suppressed, with a pulse rate exceeding 160 beats after 80 minutes during the marathon with orotic acid ingestion, compared to a pulse rate exceeding 170 beats within 50 minutes in the practice run without orotic acid ingestion.

Example 4

A male marathon runner in his twenties was used as a test subject to verify the heart rate decreasing effect during a marathon.

He did a 30 km practice run at an average pace of 04:12 min/km in sunny weather and at an ambient temperature of 16°C, and his heart rate during that time was measured in the same manner as in Example 3. The results are shown in Table 5 and Figure 4. His average heart rate during the practice run was 158 beats, and his maximum heart rate was 176 beats.

[Table 5]

|  | Total time (ratio) |
|---|---|
| Heart rate zone 5 (heart rate of 160 beats or more) | 50 min 48 s (41%) |
| Heart rate zone 4 (143-160 beats) | 1 h 11 min 52 s (58%) |
| Lower heart rate (up to 142 beats) | 1 min 30 s (1%) |

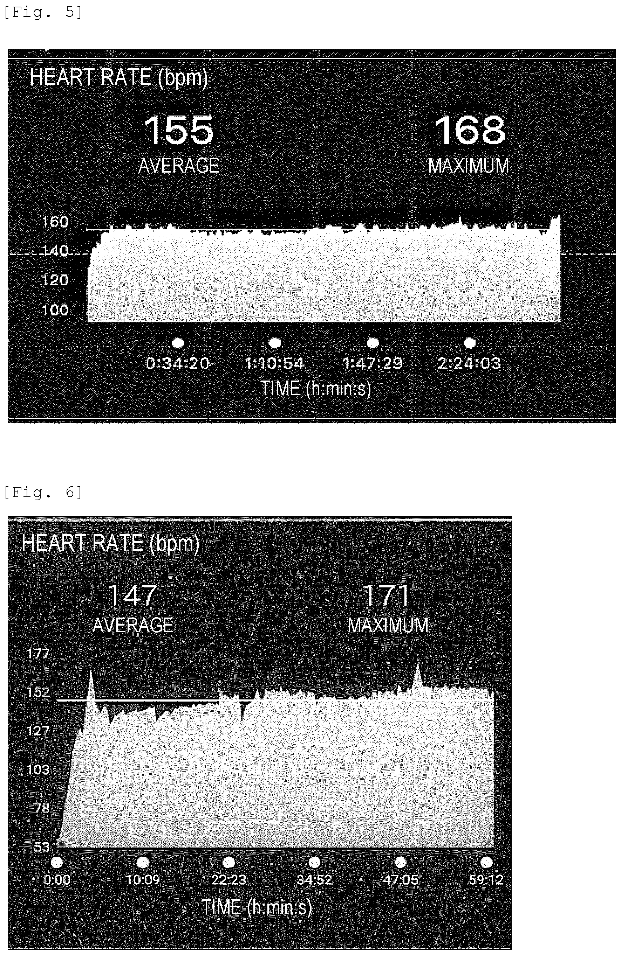

On the day of the marathon race (full marathon), the subject ingested two chewable tablets containing 200 mg of orotic acid by dissolving them in the oral cavity one hour before the start. Then, he participated in the race and completed it in 2 hours 58 minutes and 18 seconds at an average pace of 04:06 min/km. His heart rate during that time was measured in the same manner. The results are shown in Table 6 and Figure 5. The weather on the day of the race was sunny and the ambient temperature was 19°C, which were almost the same as those during the practice run. His average heart rate during the race was 155 beats, and his maximum heart rate was 168 beats.

[Table 6]

|  | Total time (ratio) |
|---|---|
| Heart rate zone 4 (165 beats or more) | 0 min 32 s (0%) |
| Heart rate zone 3 (144-164 beats) | 2 h 54 min 21 s (98%) |
| Heart rate zone 2 (124-143 beats) | 3 min 25 s (2%) |

The average pace during the practice run was 04:12 min/km, and the average pace during the marathon race was 04:06 min/km, which are almost the same pace, but when orotic acid was not ingested, 41% of the overall heart rate was in the heart rate zone 5 (160 beats and up) or higher, whereas 98% was in zone 3 (144 to 164 beats) when orotic acid was ingested, and no drift phenomenon in which the heart rate increases in the latter half was observed, which confirmed a large difference in heart rate between the case where orotic acid was ingested and the case where orotic acid was not ingested.

Example 5

A male marathon runner in his twenties was used as a test subject to verify the heart rate decreasing effect during a marathon.

The test subject completed a half marathon in 59 minutes and 33 seconds at an average pace of 02:49 min/km without ingesting orotic acid, and his heart rate during that time was measured by Garmin's GPS fitness tracker. The results are shown in Table 7 and Figure 6.

[Table 7]

|  | Total time (ratio) |
| --- | --- |
| Heart rate zone 5 (heart rate of 169 beats or more) | 0 min 14 s (0%) |
| Heart rate zone 4 (150-168 beats) | 29 min 38 s (49%) |
| Heart rate zone 3 (132-149 beats) | 27 min 42 s (46%) |
| Heart rate zone 2 (113-131 beats) | 1 min 45 s (3%) |
| Heart rate zone 1 (94-112 beats) | 0 min 14 s (0%) |

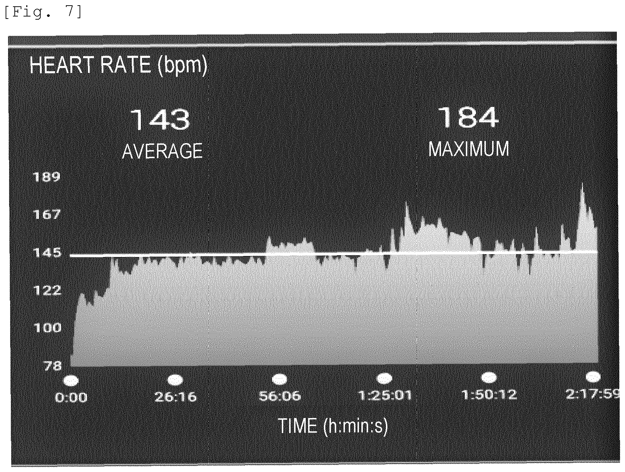

On the day of the marathon race (full marathon), the subject ingested two chewable tablets containing 200 mg of orotic acid by dissolving them in the oral cavity one hour before the start. Then, he participated in the race and completed it in 2 hours 18 minutes and 37 seconds at an average pace of 03:17 min/km. His heart rate during that time was measured in the same manner. The results are shown in Table 8 and Figure 7.

[Table 8]

|  | Total time (ratio) |
| --- | --- |
| Heart rate zone 5 (heart rate of 169 beats or more) | 1 min 49 s (1%) |
| Heart rate zone 4 (150-168 beats) | 31 min 15 s (22%) |
| Heart rate zone 3 (132-149 beats) | 1 h 33 min 59 s (68%) |
| Heart rate zone 2 (113-131 beats) | 11 min 08 s (8%) |
| Heart rate zone 1 (94-112 beats) | 0 min 26 s (0%) |

Since the average paces of the full marathon and the half marathon were different, it is not possible to simply compare them, but according to Figures 6 and 7, the average heart rate was kept lower during the full marathon with orotic acid ingestion than during the half marathon without orotic acid ingestion.

**Industrial Applicability**

By using the agent of the present invention, not only a reduction in palpitation and shortness of breath and of the load on cardiopulmonary function, but also a relaxation effect, an anti-stress effect, and the like can be expected, and the agent is useful as a safe drug capable of improving QOL in the medical field and the field of health foods such as supplements. In addition, since it can suppress an increase in heart rate during exercise, it is also useful in the sports field as a supplement for general exercising persons or athletes.

**Claims**

1. An agent for decreasing heart rate, comprising orotic acid or a salt thereof as an active ingredient.

2. An agent for preventing or ameliorating palpitation, shortness of breath or fatigue associated with an increased heart rate, wherein the agent comprises orotic acid or a salt thereof as an active ingredient.

3. The agent for preventing or ameliorating palpitation, shortness of breath or fatigue associated with an increased heart rate according to claim 2, wherein the palpitation or shortness of breath associated with an increased heart rate is caused by exercise, aging, a decrease in cardiopulmonary function or drinking.

4. An agent for suppressing an increase in heart rate during exercise, comprising orotic acid or a salt thereof as an active ingredient.

5. The agent according to any one of claims 1 to 4, which is for oral administration.

[Fig. 1]

[Fig. 2]

[Fig. 3]

HEART RATE (bpm)

161
AVERAGE

188
MAXIMUM

[Fig. 4]

HEART RATE (bpm)

158
AVERAGE

176
MAXIMUM

[Fig. 5]

[Fig. 6]

[Fig. 7]

## INTERNATIONAL SEARCH REPORT

International application No.

PCT/JP2019/042924

### A. CLASSIFICATION OF SUBJECT MATTER

A61K 31/513(2006.01)i; A61P 9/00(2006.01)i; A61P 9/04(2006.01)i; A61P 11/00(2006.01)i
FI: A61K31/513; A61P9/00; A61P9/04; A61P11/00

According to International Patent Classification (IPC) or to both national classification and IPC

### B. FIELDS SEARCHED

Minimum documentation searched (classification system followed by classification symbols)
A61K31/513; A61P9/00; A61P9/04; A61P11/00

Documentation searched other than minimum documentation to the extent that such documents are included in the fields searched

| | |
|---|---|
| Published examined utility model applications of Japan | 1922–1996 |
| Published unexamined utility model applications of Japan | 1971–2020 |
| Registered utility model specifications of Japan | 1996–2020 |
| Published registered utility model applications of Japan | 1994–2020 |

Electronic data base consulted during the international search (name of data base and, where practicable, search terms used)
JSTPlus/JMEDPlus/JST7580 (JDreamIII); CAplus/REGISTRY/MEDLINE/EMBASE/BIOSIS (STN)

### C. DOCUMENTS CONSIDERED TO BE RELEVANT

| Category* | Citation of document, with indication, where appropriate, of the relevant passages | Relevant to claim No. |
|---|---|---|
| X | MARTYNOV, A. I. et al., "Fifteen years experience of the use of magnesium preparations in patients with mitral valve prolapse." (English Translation, Original Russian), [Kardiologiia] (English notation, Original Russian), 2011, vol. 51, no. 6, pp. 60-65, MEDLINE, [online], STN, retrieval date 23 December 2019, PubMed, PMID: 21878073, abstract | 1-5 |
| Y | JP 2012-246280 A (KIRIN HOLDINGS COMPANY, LIMITED) 13.12.2012 (2012-12-13) claims, examples | 1-5 |

☒ Further documents are listed in the continuation of Box C.      ☒ See patent family annex.

| | |
|---|---|
| * Special categories of cited documents: | "T" later document published after the international filing date or priority date and not in conflict with the application but cited to understand the principle or theory underlying the invention |
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| 07 January 2020 (07.01.2020) | 21 January 2020 (21.01.2020) |

| Name and mailing address of the ISA/ | Authorized officer |
|---|---|
| Japan Patent Office 3-4-3, Kasumigaseki, Chiyoda-ku, Tokyo 100-8915, Japan | |
| | Telephone No. |

Form PCT/ISA/210 (second sheet) (January 2015)

**INTERNATIONAL SEARCH REPORT**

| International application No. |
| --- |
| PCT/JP2019/042924 |

C (Continuation).   DOCUMENTS CONSIDERED TO BE RELEVANT

| Category* | Citation of document, with indication, where appropriate, of the relevant passages | Relevant to claim No. |
| --- | --- | --- |
| Y | JP 2000-516937 A (WISS, Osawld) 19.12.2000 (2000-12-19) claims, page 11, paragraph [0002], examples 37-38 | 1-5 |
| Y | 佐藤信紘 ほか，飲酒肝血流、肝酸素消費、肝チトクロームの redox レベルに及ぼす影響について，肝臓，1981, vol. 22, no. 4, pp. 546-551, fig. 3, page 548, section 2., (SATO, Nobuhiro et al., "Acute effect of ethanol on hemoperfusion and oxidative metabolism in the liver in vivo", Kanzo) | 1-5 |

Form PCT/ISA/210 (continuation of second sheet) (January 2015)

INTERNATIONAL SEARCH REPORT

Information on patent family members

International application No.

PCT/JP2019/042924

| Patent Documents referred in the Report | Publication Date | Patent Family | Publication Date |
|---|---|---|---|
| JP 2012-246280 A | 13 Dec. 2012 | (Family: none) | |
| JP 2000-516937 A | 19 Dec. 2000 | US 2001/0020007 A1 claims, paragraph [0021], examples 37-38 US 2004/0043942 A1 WO 1998/008521 A1 EP 920321 A1 | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2011098896 A
- JP 2011136907 A
- JP 2012246280 A
- JP 2012126683 A
- JP 55015481 A
- WO 2016182053 A
- JP 7010235 A

**Non-patent literature cited in the description**

- Keio University's Sports Medical Research Center Bulletin. 1999, 33-39
- *Ministry of Health, Labour and Welfare's Study Panel Report on Revision of Exercise Standards and Exercise Guidelines,* March 2013